# EUROPEAN PATENT SPECIFICATION

(11) **EP 4 358 872 B1**
(45) Date of publication and mention of the grant of the patent: **04.11.2026**
(21) Application number: 22744583.0
(22) Date of filing: 22.06.2022
(51) Int. Cl.: A61B 17/12

(54) **LEFT ATRIAL APPENDAGE IMPLANT**
IMPLANTAT FÜR LINKES HERZOHR
IMPLANT D'APPENDICE AURICULAIRE GAUCHE

(30) Priority: 22.06.2021 US 202163213696 P
(43) Date of publication of application: 01.05.2024
(73) Proprietor: Boston Scientific Scimed, Inc., Maple Grove, MN 55311 (US)
(72) Inventor: DINGES, Eric, Maple Grove, Minnesota 55369 (US); DORAN, Burns P., Monticello, Minnesota 55362 (US); PEIFFER, Dennis A., Brooklyn Park, Minnesota 55443 (US); BEGUR, Anjali, Maple Grove, Minnesota 55369 (US); CHAU, Thyna M., Woodbury, Minnesota 55125 (US); SMITH, Grant Teal, Andover, Minnesota 55304 (US)
(74) Representative: Peterreins Schley
(86) International application number: PCT/US2022/034534
(87) International publication number: WO 2022/271832

(56) References cited:
- WO-A1-2022/118129
- US-A1- 2007 233 175
- US-A1- 2015 313 599
- US-A1- 2017 007 260
- US-A1- 2020 060 849

## Description

### Cross-Reference to Related Applications

This application claims the benefit of priority of U.S. Provisional Application No. 63/213,696 filed June 22, 2021.

### Technical Field

The disclosure relates generally to medical devices and more particularly to medical devices that are adapted for use in percutaneous medical procedures including implantation into the left atrial appendage (LAA) of a heart.

### Background

The left atrial appendage is a small organ attached to the left atrium of the heart. During normal heart function, as the left atrium constricts and forces blood into the left ventricle, the left atrial appendage constricts and forces blood into the left atrium. The ability of the left atrial appendage to contract assists with improved filling of the left ventricle, thereby playing a role in maintaining cardiac output. However, in patients suffering from atrial fibrillation, the left atrial appendage may not properly contract or empty, causing stagnant blood to pool within its interior, which can lead to the undesirable formation of thrombi within the left atrial appendage.

Thrombi forming in the left atrial appendage may break loose from this area and enter the blood stream. Thrombi that migrate through the blood vessels may eventually plug a smaller vessel downstream and thereby contribute to stroke or heart attack. Clinical studies have shown that the majority of blood clots in patients with atrial fibrillation originate in the left atrial appendage. As a treatment, medical devices have been developed which are deployed to close off the left atrial appendage. Of the known medical devices and methods, each has certain advantages and disadvantages. There is an ongoing need to provide alternative medical devices as well as alternative methods for manufacturing and using medical devices.

US 2020/060849 A1 relates to a medical implant including an expandable framework configured to shift between a collapsed configuration and an expanded configuration, the expandable framework comprising a plurality of interconnected struts defining a plurality of cells; and an occlusive element connected to the expandable framework and having an inner surface and an outer surface. The expandable framework includes a plurality of securement members projecting from the plurality of interconnected struts. One of the inner surface or the outer surface of the occlusive element may be in contact with the plurality of interconnected struts, and the other of the inner surface and the outer surface not in contact with the plurality of interconnected struts may lie against an opposing surface of each of the plurality of securement members. A tip portion of the plurality of securement members may not extend radially outward of the plurality of interconnected struts.

US 2017/007260 A1 relates to various devices, assemblies and kits for creating vascular occlusions and various methods for creating vascular occlusions using such devices, assemblies and kits.

US 2007/233175 A1 relates to a device for filtering emboli from blood flowing through a lumen defined by the walls of a vessel in a patient's body, comprising: a filter element being expandable from a collapsed configuration when the filter element is restrained to an expanded configuration when the filter element is unrestrained, and an elongate support member, the filter being carried on a portion of the elongate support member, wherein the filter element has proximal and distal portions and a central portion, the filter element having a shape in the expanded configuration which defines a cavity having a proximal facing opening, and wherein the proximal portion of the filter element comprises a radiopaque element in the form of a discontinuous loop extending around a portion of a perimeter of the proximal facing opening, the discontinuous loop having a gap and the gap being proximate to the elongate support member.

US 2015/313599 A1 relates to an implantable medical device for sealing and repairing defects in a body tissue or for creating an anastomosis which includes a frame and a covering material. The frame includes a single continuously wound wire that defines an apposition portion, a defect-occupying portion, and a sealing portion. The tissue-sealing and anastomosis devices are well-suited for use in the GI tract including the small bowel and colon. A two-part frame construct facilitates independent tailoring of apposition forces and radial forces exerted on tissues by the two-part frame.

### Summary

The invention is defined in the appended claims. In particular, the present invention pertains to an implant as defined in claim 1 and a system as defined in claim 7. Additional advantageous aspects of the invention are defined in the dependent claims.

Aspects, embodiments and examples disclosed herein which do not fall within the scope of the appended claims do not form part of the invention and are merely provided for illustrative purposes.

In one example, an implant for occluding a left atrial appendage may comprise an expandable framework configured to shift between a collapsed configuration and an expanded configuration, wherein the expandable framework includes a proximal hub and a distal hub, wherein a longitudinal axis of the expandable framework extends from the proximal hub to the distal hub, and a radiopaque marker positioned longitudinally between the proximal hub and the distal hub in the expanded configuration.

In addition or alternatively to any example disclosed herein, the radiopaque marker is oriented longitudinally in the expanded configuration.

In addition or alternatively to any example disclosed herein, the radiopaque marker includes a body portion, a proximal leg, a distal leg, a first lateral tab, and a second lateral tab. The body portion is disposed against an exterior surface of the expandable framework.

In addition or alternatively to any example disclosed herein, the proximal leg and the distal leg extend radially inwardly toward an interior of the expandable framework.

In addition or alternatively to any example disclosed herein, the first lateral tab and the second lateral tab wrap around a portion of the expandable framework.

In addition or alternatively to any example disclosed herein, the radiopaque marker includes a tubular member disposed radially inward of and secured against an inner surface of the expandable framework.

In addition or alternatively to any example disclosed herein, the radiopaque marker includes a flat plate disposed radially inward of and secured against an inner surface of the expandable framework.

In addition or alternatively to any example disclosed herein, the implant may further comprise an occlusive element disposed over at least a portion of the expandable framework.

In addition or alternatively to any example disclosed herein, the radiopaque marker includes a flattened element at least partially embedded within the occlusive element.

In addition or alternatively to any example disclosed herein, the radiopaque marker includes a plurality of radiopaque markers spaced apart about a circumference of the expandable framework.

In addition or alternatively to any example disclosed herein, a system for occluding a left atrial appendage may comprise a delivery sheath and core wire slidably disposed within a lumen of the delivery sheath and an implant for occluding a left atrial appendage releasably securable to a distal end of the core wire. The implant may comprise an expandable framework configured to shift between a collapsed configuration and an expanded configuration, wherein the expandable framework includes a proximal hub and a distal hub, wherein a longitudinal axis of the expandable framework extends from the proximal hub to the distal hub, and a radiopaque marker positioned longitudinally between the proximal hub and the distal hub in the expanded configuration.

In addition or alternatively to any example disclosed herein, the radiopaque marker is formed from a different material than the expandable framework.

In addition or alternatively to any example disclosed herein, the radiopaque marker has a different density than the expandable framework.

In addition or alternatively to any example disclosed herein, the radiopaque marker includes a body portion, a proximal leg, and a distal leg. The body portion is disposed against an exterior surface of the expandable framework. The proximal leg and the distal leg extend radially inwardly toward an interior of the expandable framework. When the implant is being moved into the lumen, the proximal leg engages the delivery sheath to urge the expandable framework radially inward and away from the delivery sheath.

In addition or alternatively to any example disclosed herein, a system for occluding a left atrial appendage may comprise a delivery sheath and a core wire slidably disposed within a lumen of the delivery sheath and an implant for occluding a left atrial appendage releasably securable to a distal end of the core wire. The implant may comprise an expandable framework configured to shift between a collapsed configuration and an expanded configuration, wherein the expandable framework includes a proximal hub and a distal hub, wherein a longitudinal axis of the expandable framework extends from the proximal hub to the distal hub, a first radiopaque marker positioned longitudinally between the proximal hub and the distal hub in the expanded configuration, a second radiopaque marker positioned longitudinally between the proximal hub and the distal hub in the expanded configuration, and a third radiopaque marker positioned longitudinally between the proximal hub and the distal hub in the expanded configuration. The first radiopaque marker, the second radiopaque marker, and the third radiopaque marker may define a plane for positioning the expandable framework relative to an ostium of the left atrial appendage in the expanded configuration.

In addition or alternatively to any example disclosed herein, the first, second, and third radiopaque markers each includes a body portion, a proximal leg, and a distal leg. The body portion is disposed against an exterior surface of the expandable framework. The proximal leg and the distal leg extend radially inwardly toward an interior of the expandable framework. When the implant is being moved into the lumen, the proximal leg engages the delivery sheath to urge the expandable framework radially inward and away from the delivery sheath.

In addition or alternatively to any example disclosed herein, the first, second, and third radiopaque markers each further includes a first lateral tab and a second lateral tab. The first lateral tab and the second lateral tab wrap around a portion of the expandable framework.

In addition or alternatively to any example disclosed herein, at least a portion of the second lateral tab overlaps the first lateral tab.

In addition or alternatively to any example disclosed herein, at least a portion of the second lateral tab extends radially inward closer to the longitudinal axis than the first lateral tab.

In addition or alternatively to any example disclosed herein, the first, second, and third radiopaque markers each define a lateral extent and a longitudinal extent, the longitudinal extent being greater than the lateral extent. The longitudinal extent is oriented longitudinally with respect to the expandable framework.

The above summary of some embodiments, aspects, and/or examples is not intended to describe each embodiment or every implementation of the present disclosure. The figures and the detailed description more particularly exemplify aspects of these embodiments.

### Brief Description of the Drawings

The disclosure may be more completely understood in consideration of the following detailed description in connection with the accompanying drawings, in which:
FIGS. 1-2 are side views of an example system for occluding a left atrial appendage;
FIGS. 3-4 illustrate selected aspects of an implant for occluding a left atrial appendage;
FIG. 5 illustrates the implant of FIGS. 3-4 disposed within a left atrial appendage;
FIG. 6 illustrates aspects of a radiopaque marker of the implant of FIG. 3;
FIG. 7 is a cross-sectional view illustrating aspects of the radiopaque marker of FIG. 6;
FIG. 8 is a side view illustrating aspects of the radiopaque marker of FIG. 6;
FIG. 9 illustrates selected aspects of the implant of FIG. 3 being moved into the outer sheath of FIGS. 1-2;
FIG. 10 illustrates an alternative configuration of a radiopaque marker for the implant of FIG. 3;
FIG. 11 illustrates an alternative configuration of a radiopaque marker for the implant of FIG. 3; and
FIGS. 12-13 illustrate an alternative configuration of a radiopaque marker for the implant of FIG. 3.

While aspects of the disclosure are amenable to various modifications and alternative forms, examples are shown in the drawings and described herein. It should be understood, however, that the intention is not to limit aspects of the disclosure to the particular embodiments described. On the contrary, the disclosure shall cover all modifications, equivalents, and alternatives falling within the scope thereof.

### Detailed Description

The following description should be read with reference to the drawings, which are not necessarily to scale, wherein like reference numerals indicate like elements throughout the several views. The detailed description and drawings are intended to illustrate but not limit the present disclosure. Those skilled in the art will recognize that the various elements described and/or shown may be arranged in various combinations and configurations without departing from the scope of the disclosure. The detailed description and drawings illustrate example embodiments of the disclosure. However, in the interest of clarity and ease of understanding, while every feature and/or element may not be shown in each drawing, the feature(s) and/or element(s) may be understood to be present regardless, unless otherwise specified.

For the following defined terms, these definitions shall be applied, unless a different definition is given in the claims or elsewhere in this specification.

All numeric values are herein assumed to be modified by the term "about," whether or not explicitly indicated. The term "about", in the context of numeric values, generally refers to a range of numbers that one of skill in the art would consider equivalent to the recited value (e.g., having the same function or result). In many instances, the term "about" may include numbers that are rounded to the nearest significant figure. Other uses of the term "about" (e.g., in a context other than numeric values) may be assumed to have their ordinary and customary definition(s), as understood from and consistent with the context of the specification, unless otherwise specified.

The recitation of numerical ranges by endpoints includes all numbers within that range, including the endpoints (e.g., 1 to 5 includes 1, 1.5, 2, 2.75, 3, 3.80, 4, and 5).

Although some suitable dimensions, ranges, and/or values pertaining to various components, features and/or specifications are disclosed, one of skill in the art, incited by the present disclosure, would understand desired dimensions, ranges, and/or values may deviate from those expressly disclosed.

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise. It is to be noted that in order to facilitate understanding, certain features of the disclosure may be described in the singular, even though those features may be plural or recurring within the disclosed embodiment(s). Each instance of the features may include and/or be encompassed by the singular disclosure(s), unless expressly stated to the contrary. For simplicity and clarity purposes, not all elements of the present disclosure are necessarily shown in each figure or discussed in detail below. However, it will be understood that the following discussion may apply equally to any and/or all of the components for which there are more than one, unless explicitly stated to the contrary. Additionally, not all instances of some elements or features may be shown in each figure for clarity.

Relative terms such as "proximal", "distal", "advance", "retract", variants thereof, and the like, may be generally considered with respect to the positioning, direction, and/or operation of various elements relative to a user/operator/manipulator of the device, wherein "proximal" and "retract" indicate or refer to closer to or toward the user and "distal" and "advance" indicate or refer to farther from or away from the user. In some instances, the terms "proximal" and "distal" may be arbitrarily assigned in an effort to facilitate understanding of the disclosure, and such instances will be readily apparent to the skilled artisan. Other relative terms, such as "upstream", "downstream", "inflow", and "outflow" refer to a direction of fluid flow within a lumen, such as a body lumen, a blood vessel, or within a device. Still other relative terms, such as "axial", "circumferential", "longitudinal", "lateral", "radial", etc. and/or variants thereof generally refer to direction and/or orientation relative to a central longitudinal axis of the disclosed structure or device.

The term "extent" may be understood to mean a greatest measurement of a stated or identified dimension, unless the extent or dimension in question is preceded by or identified as a "minimum", which may be understood to mean a smallest measurement of the stated or identified dimension. For example, "outer extent" may be understood to mean an outer dimension, "radial extent" may be understood to mean a radial dimension, "longitudinal extent" may be understood to mean a longitudinal dimension, etc. Each instance of an "extent" may be different (e.g., axial, longitudinal, lateral, radial, circumferential, etc.) and will be apparent to the skilled person from the context of the individual usage. Generally, an "extent" may be considered a greatest possible dimension measured according to the intended usage, while a "minimum extent" may be considered a smallest possible dimension measured according to the intended usage. In some instances, an "extent" may generally be measured orthogonally within a plane and/or cross-section, but may be, as will be apparent from the particular context, measured differently - such as, but not limited to, angularly, radially, circumferentially (e.g., along an arc), etc.

The terms "monolithic" and "unitary" shall generally refer to an element or elements made from or consisting of a single structure or base unit/element. A monolithic and/or unitary element shall exclude structure and/or features made by assembling or otherwise joining multiple discrete structures or elements together.

It is noted that references in the specification to "an embodiment", "some embodiments", "other embodiments", etc., indicate that the embodiment(s) described may include a particular feature, structure, or characteristic, but every embodiment may not necessarily include the particular feature, structure, or characteristic. Moreover, such phrases are not necessarily referring to the same embodiment. Further, when a particular feature, structure, or characteristic is described in connection with an embodiment, it would be within the knowledge of one skilled in the art to effect the particular feature, structure, or characteristic in connection with other embodiments, whether or not explicitly described, unless clearly stated to the contrary. That is, the various individual elements described below, even if not explicitly shown in a particular combination, are nevertheless contemplated as being combinable or arrangeable with each other to form other additional embodiments or to complement and/or enrich the described embodiment(s), as would be understood by one of ordinary skill in the art.

For the purpose of clarity, certain identifying numerical nomenclature (e.g., first, second, third, fourth, etc.) may be used throughout the description and/or claims to name and/or differentiate between various described and/or claimed features. It is to be understood that the numerical nomenclature is not intended to be limiting and is exemplary only. In some embodiments, alterations of and deviations from previously used numerical nomenclature may be made in the interest of brevity and clarity. That is, a feature identified as a "first" element may later be referred to as a "second" element, a "third" element, etc. or may be omitted entirely, and/or a different feature may be referred to as the "first" element. The meaning and/or designation in each instance will be apparent to the skilled practitioner.

The following figures illustrate selected components and/or arrangements of an implant for occluding the left atrial appendage, a system for occluding the left atrial appendage, and/or methods of using the implant and/or the system. It should be noted that in any given figure, some features may not be shown, or may be shown schematically, for simplicity. Additional details regarding some of the components of the implant and/or the system may be illustrated in other figures in greater detail. While discussed in the context of occluding the left atrial appendage, the implant and/or the system may also be used for other interventions and/or percutaneous medical procedures within a patient. Similarly, the devices and methods described herein with respect to percutaneous deployment may be used in other types of surgical procedures, as appropriate. For example, in some examples, the devices may be used in a non-percutaneous procedure. Devices and methods in accordance with the disclosure may also be adapted and configured for other uses within the anatomy.

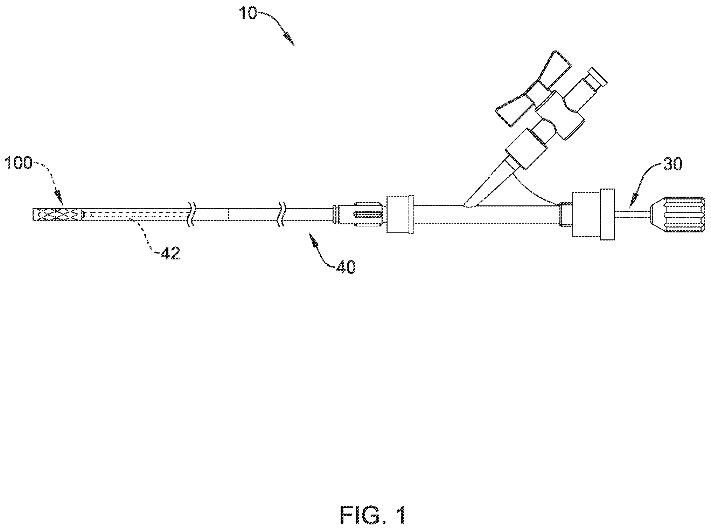

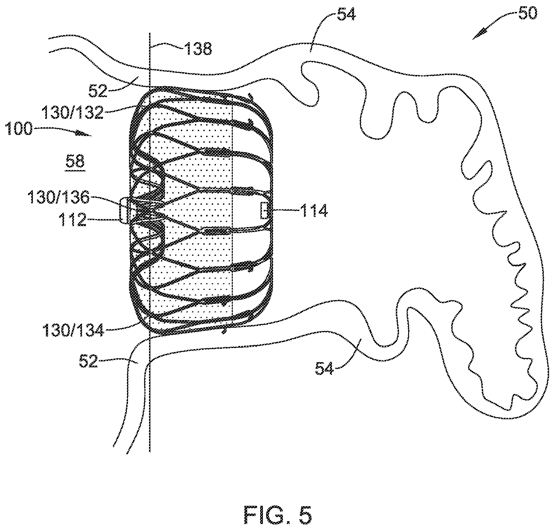

FIGS. 1-2 illustrate selected components and/or arrangements of a system 10 for occluding a left atrial appendage 50 (e.g., FIG. 5). It should be noted that in any given figure, some features of the system 10 may not be shown, or may be shown schematically, for simplicity. Additional details regarding some of the components of the system 10 may be illustrated in other figures in greater detail. The system 10 may be used to percutaneously deliver and/or deploy a variety of medical implants (e.g., a cardiovascular medical implant, an occlusive medical implant, a replacement heart valve implant, etc.) to one or more locations within the anatomy, including but not limited to, in some embodiments, the heart.

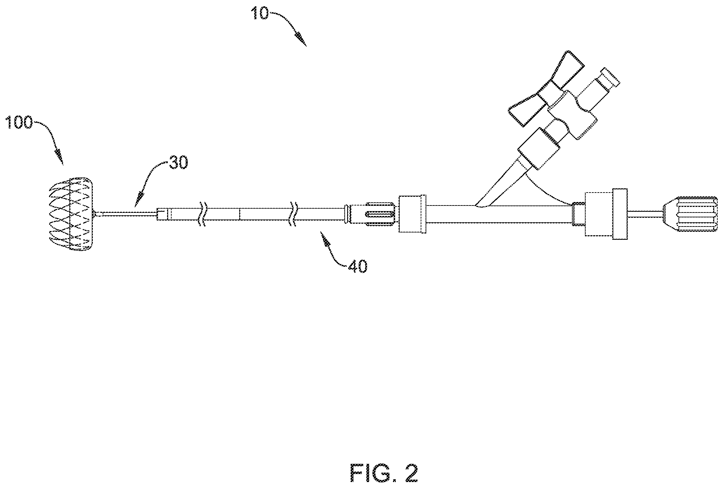

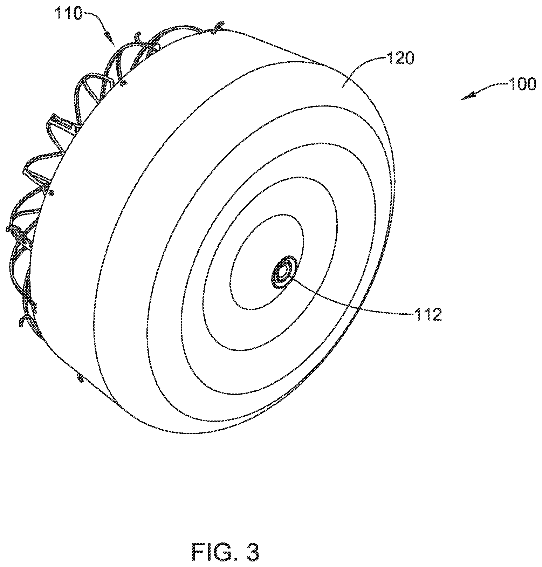

The system 10 of the claimed invention includes a delivery sheath 40 having a lumen 42 extending from a proximal opening to a distal opening, a core wire 30 slidably disposed within the lumen 42, and an implant 100 for occluding the left atrial appendage 50 (e.g., FIG. 5). The implant 100 includes an expandable framework 110 (e.g., FIGS. 3-4) configured to shift between a collapsed configuration (e.g., FIG. 1), wherein the implant 100 is disposed within the lumen 42 proximate the distal opening in the collapsed configuration, and an expanded configuration (e.g., FIG. 2), wherein the implant 100 and/or the expandable framework 110 is configured to shift between the collapsed configuration and the expanded configuration when the implant 100 is disposed distal of the distal opening of the lumen 42 and/or the delivery sheath 40, and/or when the implant 100 is unconstrained by the delivery sheath 40. In at least some embodiments, the expandable framework 110 may be self-biased toward the expanded configuration.

The implant 100 may be disposed at and/or releasably securable to a distal portion and/or a distal end of the core wire 30. The core wire 30 may be slidably and/or rotatably disposed within the lumen 42 of the delivery sheath 40. In some embodiments, a proximal end of the core wire 30 may extend proximally of a proximal end of the delivery sheath 40 and/or the proximal opening of the lumen 42 for manual manipulation by a clinician or practitioner. In some embodiments, the implant 100 may be removably attached, joined, secured, or otherwise connected to the distal end of the core wire 30. The core wire 30 may be configured to and/or may be capable of axially translating the implant 100 relative to the delivery sheath 40. The delivery sheath 40 and/or the core wire 30 may have a selected level of axial stiffness and/or pushability characteristics while also having a selected level of flexibility to permit navigation through the patient's vasculature.

Some suitable, but non-limiting, examples of materials for the system 10, the core wire 30, the delivery sheath 40, and/or the implant 100, etc. are discussed below. It is contemplated that any and/or all example implants disclosed herein may be used in accordance with and/or be associated with the example system 10 described above.

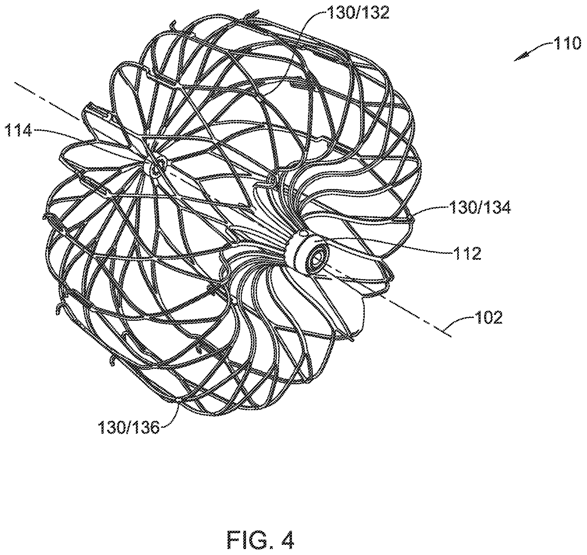

Turning now to FIGS. 3 and 4, the implant 100 of the claimed invention comprises an expandable framework 110 configured to shift along a longitudinal axis 102 (e.g., FIG. 4) between the collapsed configuration and the expanded configuration. In the collapsed configuration, the expandable framework 110 may be axially elongated and/or radially compressed. In the expanded configuration, the expandable framework 110 may be axially shortened and/or radially expanded. The expandable framework 110 may comprise a plurality of interconnected struts defining a plurality of cells. In some embodiments, the plurality of cells may be a plurality of closed cells. In some embodiments, the plurality of cells may be a plurality of open cells. In some embodiments, the plurality of cells may include a plurality of open cells and a plurality of closed cells in various combinations and/or arrangements. In some embodiments, the plurality of interconnected struts may converge, join, and/or connect at intersections or nodes.

The plurality of interconnected struts may be formed and/or cut from a tubular member. In some embodiments, the plurality of interconnected struts may be integrally formed and/or cut from a unitary member. In some embodiments, the plurality of interconnected struts may be integrally formed and/or cut from a unitary tubular member and subsequently formed and/or heat set to a desired shape in the expanded configuration. In some embodiments, the plurality of interconnected struts may be integrally formed and/or cut from a unitary flat member or sheet, and then rolled or formed into a tubular structure and subsequently formed and/or heat set to the desired shape in the expanded configuration. Some exemplary means and/or methods of making and/or forming the plurality of interconnected struts include laser cutting, machining, punching, stamping, electro discharge machining (EDM), chemical dissolution, etc. Other means and/or methods are also contemplated.

In some embodiments, the expandable framework 110 may be compliant and substantially conform to and/or be in sealing engagement with the shape and/or geometry of a lateral wall of the left atrial appendage 50 (e.g., FIG. 5) in the expanded configuration. In some embodiments, the implant 100 may expand to a size, extent, or shape less than or different from a maximum unconstrained extent, as determined by the surrounding tissue and/or lateral wall of the left atrial appendage 50. In some embodiments, reducing a thickness of various elements of the expandable framework 110 may increase the flexibility and compliance of the expandable framework 110 and/or the implant 100, thereby permitting the expandable framework 110 and/or the implant 100 to conform to the tissue around it, rather than forcing the tissue to conform to the expandable framework 110 and/or the implant 100. In some embodiments, the expandable framework 110 and/or the implant 100 may be stronger and/or less compliant, and thus the expandable framework 110 and/or the implant 100 may force the tissue of the left atrial appendage 50 to conform to the expandable framework 110 and/or the implant 100. Other configurations are also contemplated.

According to the claimed invention, the expandable framework 110 includes a proximal hub 112 and a distal hub 114, as seen in FIG. 4. A longitudinal axis 102 of the expandable framework 110 extends from the proximal hub 112 to the distal hub 114. In at least some embodiments, the proximal hub 112 and/or the distal hub 114 may be centered on and/or coaxial with the longitudinal axis 102. The plurality of interconnected struts may be joined together at and/or fixedly attached to the proximal hub 112 and/or the distal hub 114. The proximal hub 112 may be configured to releasably connect, secure, and/or attach the implant 100 and/or the expandable framework 110 to the core wire 30 (e.g., FIGS. 1-2). In some embodiments, the proximal hub 112 may include internal threads configured to rotatably and/or threadably engage an externally threaded distal end of the core wire 30. Other configurations for releasably securing the implant 100 to the core wire 30 are also contemplated.

Returning to FIG. 3, in some embodiments, the implant 100 may optionally include an occlusive element 120 connected to, disposed on, disposed over, disposed about, and/or disposed radially outward of at least a portion of the expandable framework 110 and/or the plurality of interconnected struts. In some embodiments, the occlusive element 120 may be attached to the proximal hub 112 and/or may be attached to the expandable framework at the proximal hub 112. In some embodiments, the occlusive element 120 may extend radially outward from and/or may extend distally from the proximal hub 112. In some embodiments, the occlusive element 120 may be attached and/or secured to the expandable framework 110 at a plurality of discrete locations.

In some embodiments, the occlusive element 120 may include a membrane, a fabric, a mesh, a tissue element, or another suitable construction. In some embodiments, the occlusive element 120 may be porous. In some embodiments, the occlusive element 120 may be non-porous. In some embodiments, the occlusive element 120 may be permeable or impermeable to blood and/or other fluids, such as water. In some embodiments, the occlusive element 120 may be designed, sized, and/or configured to prevent thrombus and/or embolic material from passing out of the left atrial appendage 50 into the left atrium 58 (e.g., FIG. 5) and/or the patient's bloodstream. In some embodiments, the occlusive element 120 (e.g., the membrane, the fabric, or the tissue element, etc.) promotes endothelization after implantation, thereby effectively removing the target site (e.g., the left atrial appendage 50, etc.) from the patient's circulatory system. Some suitable, but non-limiting, examples of materials for the occlusive element 120 are discussed below.

Turning now to FIGS. 4 and 5, the implant 100 of the claimed invention includes a radiopaque marker 130. In some embodiments, the radiopaque marker 130 may include a plurality of radiopaque markers 130 spaced apart about a circumference of the expandable framework 110. In some embodiments, the implant 100 may include a first radiopaque marker 132, a second radiopaque marker 134, and a third radiopaque marker 136. More or fewer radiopaque markers 130 are also contemplated. For example, the implant 100 may include four, five, six, eight, ten, twelve, fifteen, eighteen, etc. radiopaque markers. In some embodiments, the plurality of radiopaque markers 130 may include at least the first radiopaque marker 132, the second radiopaque marker 134, and the third radiopaque marker 136. In some embodiments, the plurality of radiopaque markers 130 may be equally spaced apart about the circumference of the expandable framework 110. For example, the plurality of radiopaque markers 130 may be spaced apart about 120 degrees from each other. Other spacing is also contemplated, and in some embodiments, spacing may be dependent upon how many radiopaque markers 130 are present. For example, the plurality of radiopaque markers 130 may be spaced apart about 180 degrees, about 120 degrees, about 90 degrees, about 72 degrees, about 60 degrees, about 45 degrees, about 30 degrees, etc. In some embodiments, the plurality of radiopaque markers 130 may be variably spaced apart about the circumference of the expandable framework 110. Other configurations are also contemplated.

As may be seen in the side, partial-cross-sectional view of FIG. 5, the implant 100 may be positioned within the left atrial appendage 50. The left atrial appendage 50 may have a complex geometry and/or irregular surface area. The left atrial appendage 50 is attached to and in fluid communication with the left atrium 58 of the patient's heart. Those skilled in the art will recognize that the illustrated left atrial appendage 50 is merely one of many possible shapes and sizes for the left atrial appendage 50, which may vary from patient to patient. Those of skill in the art will also recognize that the medical devices and methods disclosed herein may be adapted for various sizes and shapes of the left atrial appendage 50, as necessary. The left atrial appendage 50 may include a generally longitudinal axis arranged along a depth of a main body of the left atrial appendage 50 defined by a lateral wall 54. The left atrial appendage 50 may include the ostium 52 forming a proximal mouth opening into the left atrium 58. In some embodiments, a lateral extent of the ostium 52 and/or the lateral wall 54 may be smaller or less than a depth of the main body, or a depth of the main body may be greater than a lateral extent of the ostium 56 and/or the lateral wall 54. In some embodiments, the left atrial appendage 50 may include a distalmost region formed or arranged as a tail-like element associated with a distal portion of the main body. In some embodiments, the distalmost region may protrude radially or laterally away from the main body.

The radiopaque marker 130 and/or the plurality of radiopaque markers 130 may be positioned longitudinally between the proximal hub 112 and the distal hub 114 in the expanded configuration. For example, the radiopaque marker 130 and/or the plurality of radiopaque markers 130 may be positioned distal of the proximal hub 112 and proximal of the distal hub 114. In another example, a plane oriented perpendicular to the longitudinal axis 102 and extending through the radiopaque marker 130 and/or the plurality of radiopaque markers 130 may be positioned distal of the proximal hub 112 and proximal of the distal hub 114. In some embodiments, the first radiopaque marker 132 may be positioned longitudinally between the proximal hub 112 and the distal hub 114 in the expanded configuration, the second radiopaque marker 134 may be positioned longitudinally between the proximal hub 112 and the distal hub 114 in the expanded configuration, and the third radiopaque marker 136 may be positioned longitudinally between the proximal hub 112 and the distal hub 114 in the expanded configuration. For example, the first radiopaque marker 132, the second radiopaque marker 134, and the third radiopaque marker 136 may be positioned distal of the proximal hub 112 and proximal of the distal hub 114. In another example, a plane oriented perpendicular to the longitudinal axis 102 and extending through the first radiopaque marker 132, the second radiopaque marker 134, and/or the third radiopaque marker 136 may be positioned distal of the proximal hub 112 and proximal of the distal hub 114. In some embodiments, the first radiopaque marker 132, the second radiopaque marker 134, and the third radiopaque marker 136 may define a plane 138 for positioning the expandable framework 110 in, at, and/or relative to an ostium 52 of the left atrial appendage 50 in the expanded configuration.

In some embodiments, the radiopaque marker 130 and/or the plurality of radiopaque markers 130 may be used as fiducial markers to watch their position under fluoroscopy (or another type of imaging) relative to some background anatomy (e.g., ribs, etc.) when a tug test is performed to assess anchoring. If the radiopaque marker 130 and/or the plurality of radiopaque markers 130 are positioned at a starting location and end in another location, the imaging will show that the implant 100 and/or the expandable framework 110 has shifted. Alternatively, if the radiopaque marker 130 and/or the plurality of radiopaque markers 130 remain fixed at the starting location after the test, the implant 100 and/or the expandable framework 110 has not moved and in anchored securely.

In some embodiments, the plurality of radiopaque markers 130 may be used as deployment aids. For example, as the implant 100 and/or the expandable framework 110 exits the delivery sheath 40 and/or when the expandable framework 110 begins to shift to the expanded configuration, the plurality of radiopaque markers 130 may begin to move apart from each other under fluoroscopy (or another type of imaging) and indicate that the implant 100 and/or the expandable framework 110 is opening and/or nearing the final stages of deployment. In some embodiments, the plurality of radiopaque markers 130 may help the physician locate the implant 100 during deployment such that an idealized position may be achieved during initial deployment.

In some embodiments, the plurality of radiopaque markers 130 may be used to take size measurements for compression under fluoroscopy (or another type of imaging). In some embodiments, the plurality of radiopaque markers 130 may be used to ensure sealing of the implant 100 against the ostium 52 and/or the lateral wall 54 of the left atrial appendage 50 when a contrast puff is also used. Other configurations and/or uses are also contemplated.

As noted above, in order to facilitate understanding, certain features of the disclosure may be described in the singular, even though those features may be plural or recurring within the disclosed embodiment(s). Each instance of the features may include and/or be encompassed by the singular disclosure(s), unless expressly stated to the contrary. For simplicity and clarity purposes, not all elements of the present disclosure are necessarily shown in each figure or discussed in detail below. However, it will be understood that the following discussion may apply equally to any and/or all of the components for which there are more than one, unless explicitly stated to the contrary. As such, any one of and/or every one of the plurality of radiopaque markers 130 and/or the first radiopaque marker 132, the second radiopaque marker 134, and the third radiopaque marker 136, etc. may be encompassed by the current disclosure.

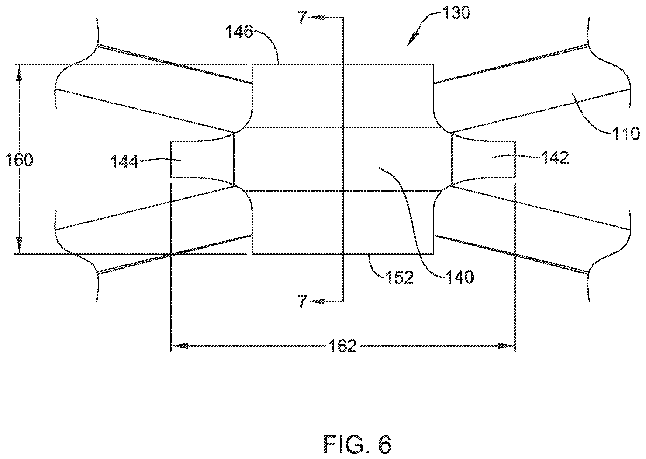

FIG. 6 illustrates one configuration of the radiopaque marker 130 (and/or the plurality of radiopaque markers 130; the first radiopaque marker 132, the second radiopaque marker 134, and the third radiopaque marker 136, etc.) as viewed from outside of the implant 100 and/or the expandable framework 110. According to the claimed invention, the radiopaque marker 130 includes a body portion 140, a proximal leg 142 and a distal leg 144, a first lateral tab 146 and a second lateral tab 152, wherein the body portion 140 is disposed and/or positioned against an exterior surface of the expandable framework 110. In some alternative embodiments, which do not fall within the scope of the claimed invention, the body portion 140 may be disposed and/or positioned against an interior surface of the expandable framework 110. Other configurations are also contemplated.

In some embodiments, the body portion 140 may be positioned at and/or against an intersection or node of the plurality of interconnected struts of the expandable framework 110. In some embodiments, the body portion 140 may be positioned at and/or against a radially outermost intersection or node of the plurality of interconnected struts of the expandable framework 110. In some embodiments, the body portion 140 may be positioned at and/or against a proximalmost intersection or node of the plurality of interconnected struts of the expandable framework 110. Other configurations are also contemplated.

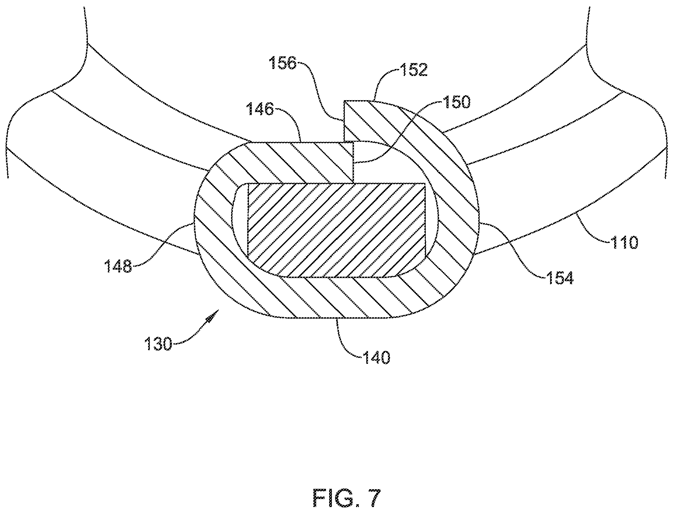

As seen in the cross-sectional view of FIG. 7, the first lateral tab 146 may extend laterally from the body portion 140 in a first direction. The second lateral tab 152 may extend laterally from the body portion 140 in a second direction opposite the first direction. In some embodiments, a first medial portion 148 of the first lateral tab 146 may extend in the first direction from the body portion 140. The first medial portion 148 of the first lateral tab 146 may extend radially inward toward the longitudinal axis 102 and/or toward an interior of the implant 100 and/or the expandable framework 110. In some embodiments, a second medial portion 154 of the second lateral tab 152 may extend in the second direction from the body portion 140. The second medial portion 154 of the second lateral tab 152 may extend radially inward toward the longitudinal axis 102 and/or toward the interior of the implant 100 and/or the expandable framework 110. In some embodiments, a first free end 150 of the first lateral tab 146 may extend laterally in the second direction from the first medial portion 148 of the first lateral tab 146. In some embodiments, a second free end 156 of the second lateral tab 152 may extend laterally in the first direction from the second medial portion 154 of the second lateral tab 152. According to the claimed invention, the first lateral tab 146 and the second lateral tab 152 wrap around a portion of the expandable framework 110. For example, the first lateral tab 146 and the second lateral tab 152 may wrap around an intersection or node of the plurality of interconnected struts of the expandable framework 110. In some embodiments, at least a portion of the second free end 156 of the second lateral tab 152 may overlap the first free end 150 of the first lateral tab 146. In some embodiments, at least a portion of the second lateral tab 152 may extend radially inwardly closer to the longitudinal axis 102 than the first lateral tab 146. In some embodiments, the second free end 156 of the second lateral tab 152 may extend radially inwardly closer to the longitudinal axis 102 than the first free end 150 of the first lateral tab 146. In some embodiments, the first free end 150 of the first lateral tab 146 may abut the second free end 156 of the second lateral tab 152. In such embodiments, the first free end 150 and the second free end 156 may be substantially equidistant from the longitudinal axis 102. Other configurations are also contemplated.

Returning briefly to FIG. 6, the radiopaque marker 130 (and/or the plurality of radiopaque markers 130 and/or the first radiopaque marker 132, the second radiopaque marker 134, and the third radiopaque marker 136, etc.) may define a lateral extent 160 and a longitudinal extent 162. In at least some embodiments, the longitudinal extent 162 may be greater than the lateral extent 160. The longitudinal extent 162 may be oriented longitudinally with respect to the expandable framework 110. Other configurations are also contemplated.

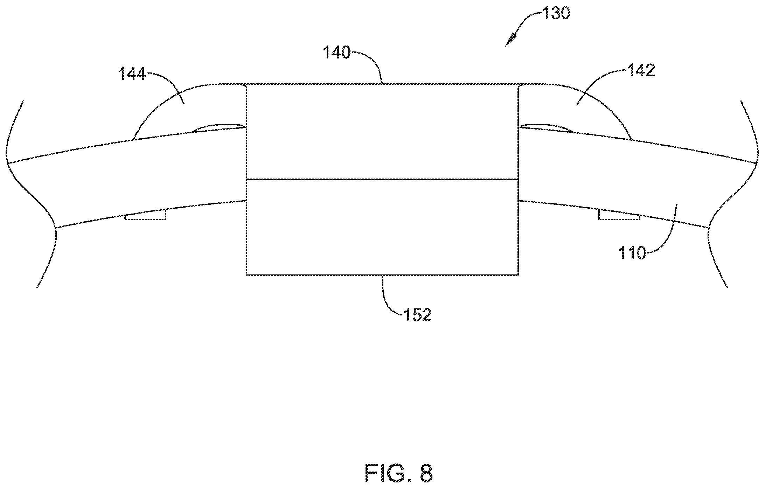

As seen in FIGS. 6 and 8, the proximal leg 142 may extend from the body portion 140 longitudinally toward a proximal end of the implant 100 and/or the expandable framework 110 and/or the proximal leg 142 may extend from the body portion 140 radially inward toward the longitudinal axis 102 and/or toward the interior of the implant 100 and/or the expandable framework 110. The distal leg 144 may extend from the body portion 140 longitudinally toward a distal end of the implant 100 and/or the expandable framework 110 and/or the distal leg 144 may extend from the body portion 140 radially inward toward the longitudinal axis 102 and/or toward the interior of the implant 100 and/or the expandable framework 110.

In some embodiments, the proximal leg 142 may extend from the body portion 140 radially inward toward the longitudinal axis 102 and/or toward the interior of the implant 100 and/or the expandable framework 110 until a free end of the proximal leg 142 is disposed radially inward of an interior surface of the expandable framework 110. In some embodiments, the proximal leg 142 may extend from the body portion 140 radially inward toward the longitudinal axis 102 and/or toward the interior of the implant 100 and/or the expandable framework 110 until the free end of the proximal leg 142 is disposed substantially flush with the interior surface of the expandable framework 110. In some embodiments, the distal leg 144 may extend from the body portion 140 radially inward toward the longitudinal axis 102 and/or toward the interior of the implant 100 and/or the expandable framework 110 until a free end of the distal leg 144 is disposed radially inward of an interior surface of the expandable framework 110. In some embodiments, the distal leg 144 may extend from the body portion 140 radially inward toward the longitudinal axis 102 and/or toward the interior of the implant 100 and/or the expandable framework 110 until the free end of the distal leg 144 is disposed substantially flush with the interior surface of the expandable framework 110. Other configurations are also contemplated.

In some embodiments, the radiopaque marker 130 may be made by punching and/or stamping the radiopaque marker 130 from a flat sheet or strip of material and then formed using one or more suitable methods. In some embodiments, the radiopaque marker 130 may be laser cut from a flat sheet or strip of material and then formed using one or more suitable methods. In some embodiments, the radiopaque marker 130 may be formed by die casting, injection molding, etc. In some embodiments, the radiopaque marker 130 may be cut and formed using a progressive die in a stamping machine or other similar methods. In some embodiments, the radiopaque marker 130 may be crimped onto the expandable framework 110. In some embodiments, the radiopaque marker 130 may be bonded and/or welded to the expandable framework 110. Other configurations and/or methods are also contemplated.

In some embodiments, the radiopaque marker 130 may be formed from a different material than the expandable framework 110. In some embodiments, the radiopaque marker 130 may have a different density than the expandable framework 110. In at least some embodiments, the radiopaque marker 130 may have a greater density than the expandable framework 110. Some suitable, but non-limiting, examples of materials for the radiopaque marker 130, the plurality of radiopaque markers 130, the first radiopaque marker 132, the second radiopaque marker 134, and the third radiopaque marker 136, etc., are discussed below.

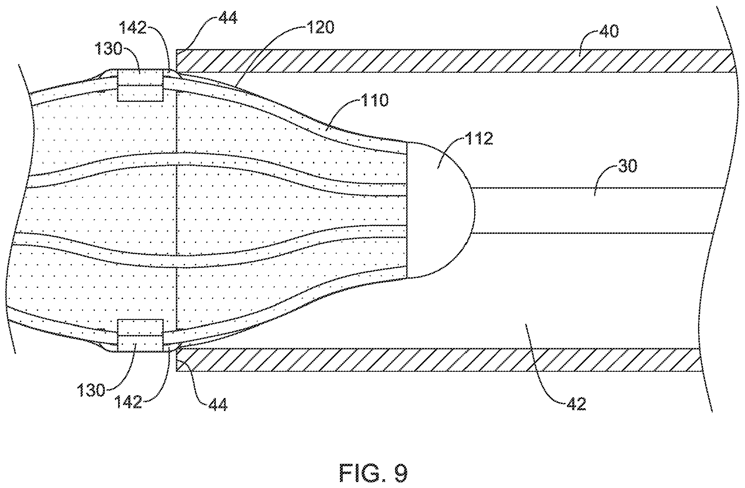

As discussed above, according to the claimed invention, the system 10 for occluding the left atrial appendage 50 includes the delivery sheath 40 having the lumen 42 extending therein and the core wire 30 slidably disposed within the lumen 42 of the delivery sheath 40. When preparing for a procedure, the implant 100 needs to be moved into the lumen 42 of the delivery sheath 40. In some instances, the implant 100 may need to be re-sheathed and/or moved back into the lumen 42 of the delivery sheath 40 in situ, such as when the implant 100 needs to be repositioned and/or removed prior to release. FIG. 9 illustrates selected aspects of the system 10 and/or the implant 100 when the implant 100 is being moved into the lumen 42 of the delivery sheath 40. All elements and/or features of the implant 100 are not shown (or are not shown completely) in order to improve clarity.

As may be seen in FIG. 9, when the implant 100 is being moved into the lumen 42 of the delivery sheath 40, the proximal leg 142 of the radiopaque marker 130 (and/or the plurality of radiopaque markers 130, the first radiopaque marker 132, the second radiopaque marker 134, and the third radiopaque marker 136, etc.) may engage a distalmost end 44 of the delivery sheath 40 and act as a ramp to guide and/or urge the expandable framework 110 radially inward and away from an inner surface of the delivery sheath 40. In at least some embodiments, the occlusive element 120 may be disposed between the radiopaque marker 130 (and/or the plurality of radiopaque markers 130, the first radiopaque marker 132, the second radiopaque marker 134, and the third radiopaque marker 136, etc.) and/or the proximal leg 142 and the distalmost end 44 of the delivery sheath 40. However, the occlusive element 120 fails to negatively affect the ramping and/or guiding function of the proximal leg 142 of the radiopaque marker 130 (and/or the plurality of radiopaque markers 130, the first radiopaque marker 132, the second radiopaque marker 134, and the third radiopaque marker 136, etc.).

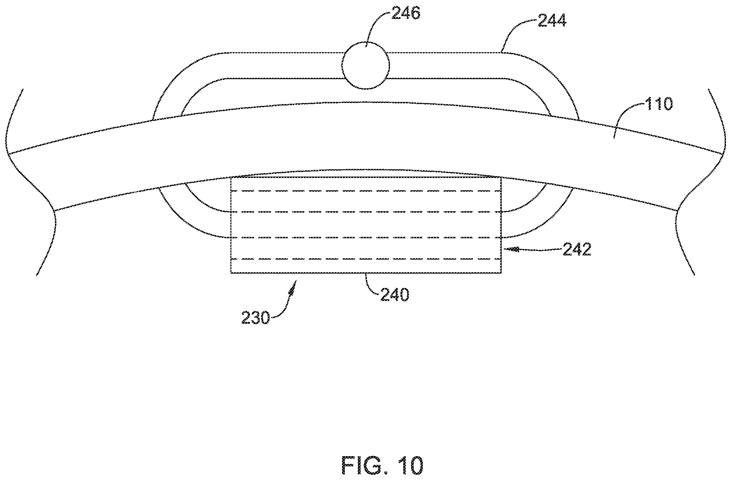

FIG. 10 illustrates an alternative configuration of a radiopaque marker 230 associated with the implant 100, wherein this alternative radiopaque marker 230 is not according to the claimed invention. In accordance with the rest of the disclosure, in some embodiments, the radiopaque marker 230 may include a plurality of radiopaque markers 230 and/or a first radiopaque marker, a second radiopaque marker, a third radiopaque marker, etc. and is discussed in the singular in the interest of brevity. The radiopaque marker 230 may include a tubular member 240 disposed radially inward of an inner surface of the expandable framework 110. In some embodiments, the radiopaque marker 230 and/or the tubular member 240 may be secured against the inner surface of the expandable framework 110. In some embodiments, the radiopaque marker 230 and/or the tubular member 240 may be positioned at and/or against an intersection or node of the plurality of interconnected struts of the expandable framework 110. In some embodiments, the radiopaque marker 230 and/or the tubular member 240 may be positioned at and/or against a radially outermost intersection or node of the plurality of interconnected struts of the expandable framework 110. In some embodiments, the radiopaque marker 230 and/or the tubular member 240 may be positioned at and/or against a proximalmost intersection or node of the plurality of interconnected struts of the expandable framework 110. Other configurations are also contemplated.

In some embodiments, the radiopaque marker 230 and/or the tubular member 240 may be secured to and/or against the expandable framework 110 using a filament 244 extending through a lumen 242 of the tubular member 240 and/or the radiopaque marker 230. In some embodiments, the filament 244 may be a suture, a wire, or other suitable element. In some embodiments, the filament 244 may be secured and/or fixed together at a securement element 246. The securement element 246 may be a knot, a weld, or another means of securing the filament 244. Other configurations are also contemplated.

In some embodiments, the radiopaque marker 230 (and/or the plurality of radiopaque markers 230 and/or the first radiopaque marker, the second radiopaque marker, and the third radiopaque marker, etc.) and/or the tubular member 240 may define a lateral extent and a longitudinal extent. In at least some embodiments, the longitudinal extent may be greater than the lateral extent. The longitudinal extent may be oriented longitudinally with respect to the expandable framework 110. Other configurations are also contemplated.

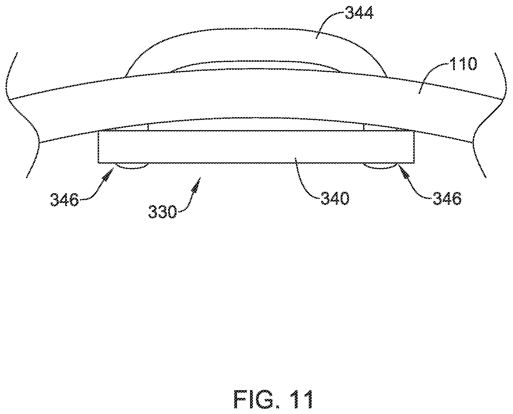

FIG. 11 illustrates an alternative configuration of a radiopaque marker 330 associated with the implant 100, wherein this alternative radiopaque marker 330 is not according to the claimed invention. In accordance with the rest of the disclosure, in some embodiments, the radiopaque marker 330 may include a plurality of radiopaque markers 330 and/or a first radiopaque marker, a second radiopaque marker, a third radiopaque marker, etc. and is discussed in the singular in the interest of brevity. The radiopaque marker 330 may include a flat plate 340 disposed radially inward of an inner surface of the expandable framework 110. In some embodiments, the radiopaque marker 330 and/or the flat plate 340 may be secured against the inner surface of the expandable framework 110. In some embodiments, the radiopaque marker 330 and/or the flat plate 340 may be positioned at and/or against an intersection or node of the plurality of interconnected struts of the expandable framework 110. In some embodiments, the radiopaque marker 330 and/or the flat plate 340 may be positioned at and/or against a radially outermost intersection or node of the plurality of interconnected struts of the expandable framework 110. In some embodiments, the radiopaque marker 330 and/or the flat plate 340 may be positioned at and/or against a proximalmost intersection or node of the plurality of interconnected struts of the expandable framework 110. Other configurations are also contemplated.

In some embodiments, the radiopaque marker 330 and/or the flat plate 340 may be secured to and/or against the expandable framework 110 using a filament 344. In some embodiments, the filament 344 may be a suture, a wire, or other suitable element. In some embodiments, the filament 344 may pass through one or more apertures in the flat plate 340. In some embodiments, the filament 344 may be secured and/or fixed to the flat plate 340 at one or more securement elements 346. The one or more securement elements 346 may be a knot, a weld, or another means of securing the filament 344. In one example, the filament 344 may be a wire that is welded to the flat plate 340 at the one or more securement elements 346. Other configurations are also contemplated.

In some embodiments, the radiopaque marker 330 (and/or the plurality of radiopaque markers 330 and/or the first radiopaque marker, the second radiopaque marker, and the third radiopaque marker, etc.) and/or the flat plate 340 may define a lateral extent and a longitudinal extent. In at least some embodiments, the longitudinal extent may be greater than the lateral extent. The longitudinal extent may be oriented longitudinally with respect to the expandable framework 110. Other configurations are also contemplated.

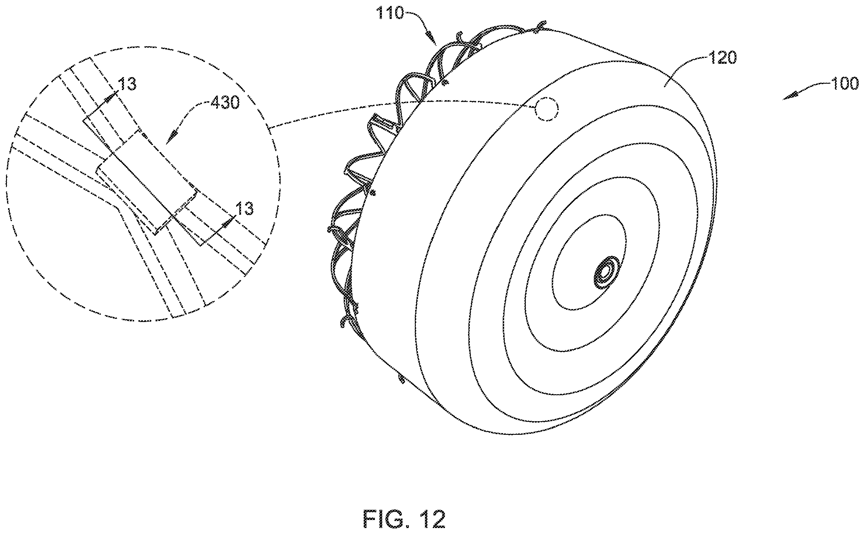

FIG. 12 illustrates an alternative configuration of a radiopaque marker 430 associated with the implant 100 and/or the occlusive element 120, wherein this alternative radiopaque marker 430 is not according to the claimed invention.

In accordance with the rest of the disclosure, in some embodiments, the radiopaque marker 430 may include a plurality of radiopaque markers 430 and/or a first radiopaque marker, a second radiopaque marker, a third radiopaque marker, etc. and is discussed in the singular in the interest of brevity. The radiopaque marker 430 may include a flattened element 440 disposed radially outward of the exterior surface of the expandable framework 110. In some embodiments, the radiopaque marker 430 and/or the flattened element 440 may be positioned at and/or over an intersection or node of the plurality of interconnected struts of the expandable framework 110. In some embodiments, the radiopaque marker 430 and/or the flattened element 440 may be positioned at and/or over a radially outermost intersection or node of the plurality of interconnected struts of the expandable framework 110. In some embodiments, the radiopaque marker 430 and/or the flattened element 440 may be positioned at and/or over a proximalmost intersection or node of the plurality of interconnected struts of the expandable framework 110. Other configurations are also contemplated.

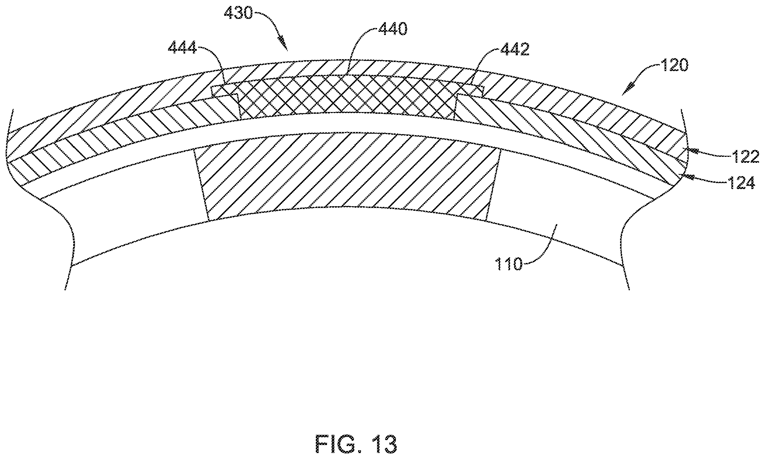

In contrast to other examples described herein, the radiopaque marker 430 and/or the flattened element 440 may be secured to and/or fixedly attached to the occlusive element 120. In some embodiments, the radiopaque marker 430 and/or the flattened element 440 may be at least partially embedded within the occlusive element 120. In some embodiments, the flattened element 440 may be partially exposed to the inside, as seen in FIG. 13, or the outside of the occlusive element 120. In some embodiments, the flattened element 440 may be completely embedded within the occlusive element 120.

In some embodiments, the occlusive element 120 may include a first layer 122 and a second layer 124. In some embodiments, at least a portion of the flattened element 440 may be disposed between the first layer 122 and the second layer 124 of the occlusive element 120. In some embodiments, the first layer 122 and the second layer 124 of the occlusive element 120 may be fixedly attached to each other. In one example, the first layer 122 of the occlusive element 120 may be bonded to the second layer 124 of the occlusive element 120. In another example, the first layer 122 of the occlusive element 120 may be welded to the second layer 124 of the occlusive element 120. Other configurations are also contemplated.

In some embodiments, an outwardly facing surface of the flattened element 440 may be engaged against, bonded to, and/or may face an inwardly facing surface of the occlusive element 120, and an inwardly facing surface of the flattened element 440 may face away from the occlusive element 120 and/or may facing toward the interior of the implant 100 and/or the expandable framework 110. Other configurations are also contemplated.

In some embodiments, the flattened element 440 may include a first flange 442 at and/or proximate a proximal end of the flattened element 440 and a second flange 444 at and/or proximate a distal end of the flattened element 440. In some embodiments, the first flange 442 and/or the second flange 444 may be embedded within the occlusive element 120 and/or may be disposed between the first layer 122 and the second layer 124 of the occlusive element 120. In some embodiments, an outwardly facing surface of the first flange 442 may face towards the first layer 122 of the occlusive element 120 and an inwardly facing surface of the first flange 442 may face towards the second layer 124 of the occlusive element 120. In some embodiments, an outwardly facing surface of the second flange 444 may face towards the first layer 122 of the occlusive element 120 and an inwardly facing surface of the second flange 444 may face towards the second layer 124 of the occlusive element 120. Other configurations are also contemplated.

In some embodiments, the first flange 442 of the flattened element 440 may be fixedly attached to the first layer 122 and/or the second layer 124 of the occlusive element 120. In some embodiments, the second flange 444 of the flattened element 440 may be fixedly attached to the first layer 122 and/or the second layer 124 of the occlusive element 120. In some embodiments, the first flange 442 of the flattened element 440 and the second flange 444 of the flattened element 440 may be fixedly attached to the first layer 122 and/or the second layer 124 of the occlusive element 120. Other configurations are also contemplated.

In some embodiments, the radiopaque marker 430 (and/or the plurality of radiopaque markers 430 and/or the first radiopaque marker, the second radiopaque marker, and the third radiopaque marker, etc.) and/or the flattened element 440 may define a lateral extent and a longitudinal extent. In at least some embodiments, the longitudinal extent may be greater than the lateral extent. The longitudinal extent may be oriented longitudinally with respect to the expandable framework 110. Other configurations are also contemplated.

The materials that can be used for the various components of the system (and/or other elements disclosed herein) and the various components thereof disclosed herein may include those commonly associated with medical devices and/or systems. For simplicity purposes, the following discussion refers to the system. However, this is not intended to limit the devices and methods described herein, as the discussion may be applied to other elements, members, components, or devices disclosed herein, such as, but not limited to, the implant, the delivery sheath, the core wire, the expandable framework, the occlusive element, etc. and/or elements or components thereof.

In some embodiments, the system and/or components thereof may be made from a metal, metal alloy, polymer (some examples of which are disclosed below), a metal-polymer composite, ceramics, combinations thereof, and the like, or other suitable material.

Some examples of suitable metals and metal alloys include stainless steel, such as 444V, 444L, and 314LV stainless steel; mild steel; nickel-titanium alloy such as linear-elastic and/or super-elastic nitinol; other nickel alloys such as nickel-chromium-molybdenum alloys (e.g., UNS: N06625 such as INCONEL^{®} 625, UNS: N06022 such as HASTELLOY^{®} C-22^{®}, UNS: N10276 such as HASTELLOY^{®} C276^{®}, other HASTELLOY^{®} alloys, and the like), nickel-copper alloys (e.g., UNS: N04400 such as MONEL^{®} 400, NICKELVAC^{®} 400, NICORROS^{®} 400, and the like), nickel-cobalt-chromium-molybdenum alloys (e.g., UNS: R44035 such as MP35-N^{®} and the like), nickel-molybdenum alloys (e.g., UNS: N10665 such as HASTELLOY^{®} ALLOY B2^{®}), other nickel-chromium alloys, other nickel-molybdenum alloys, other nickel-cobalt alloys, other nickel-iron alloys, other nickel-copper alloys, other nickel-tungsten or tungsten alloys, and the like; cobalt-chromium alloys; cobalt-chromium-molybdenum alloys (e.g., UNS: R44003 such as ELGILOY^{®}, PHYNOX^{®}, and the like); platinum enriched stainless steel; titanium; combinations thereof; and the like; or any other suitable material.

As alluded to herein, within the family of commercially available nickel-titanium or nitinol alloys, is a category designated "linear elastic" or "non-super-elastic" which, although may be similar in chemistry to conventional shape memory and super elastic varieties, may exhibit distinct and useful mechanical properties. Linear elastic and/or non-super-elastic nitinol may be distinguished from super elastic nitinol in that the linear elastic and/or non-super-elastic nitinol does not display a substantial "superelastic plateau" or "flag region" in its stress/strain curve like super elastic nitinol does. Instead, in the linear elastic and/or non-super-elastic nitinol, as recoverable strain increases, the stress continues to increase in a substantially linear, or a somewhat, but not necessarily entirely linear relationship until plastic deformation begins or at least in a relationship that is more linear than the super elastic plateau and/or flag region that may be seen with super elastic nitinol. Thus, for the purposes of this disclosure linear elastic and/or non-super-elastic nitinol may also be termed "substantially" linear elastic and/or non-super-elastic nitinol.

In some cases, linear elastic and/or non-super-elastic nitinol may also be distinguishable from super elastic nitinol in that linear elastic and/or non-super-elastic nitinol may accept up to about 2-5% strain while remaining substantially elastic (e.g., before plastically deforming) whereas super elastic nitinol may accept up to about 8% strain before plastically deforming. Both of these materials can be distinguished from other linear elastic materials such as stainless steel (that can also be distinguished based on its composition), which may accept only about 0.2 to 0.44 percent strain before plastically deforming.

In some embodiments, the linear elastic and/or non-super-elastic nickel-titanium alloy is an alloy that does not show any martensite/austenite phase changes that are detectable by differential scanning calorimetry (DSC) and dynamic metal thermal analysis (DMTA) analysis over a large temperature range. For example, in some embodiments, there may be no martensite/austenite phase changes detectable by DSC and DMTA analysis in the range of about -60 degrees Celsius (°C) to about 120 °C in the linear elastic and/or non-super-elastic nickel-titanium alloy. The mechanical bending properties of such material may therefore be generally inert to the effect of temperature over this very broad range of temperature. In some embodiments, the mechanical bending properties of the linear elastic and/or non-super-elastic nickel-titanium alloy at ambient or room temperature are substantially the same as the mechanical properties at body temperature, for example, in that they do not display a super-elastic plateau and/or flag region. In other words, across a broad temperature range, the linear elastic and/or non-super-elastic nickel-titanium alloy maintains its linear elastic and/or non-super-elastic characteristics and/or properties.

In some embodiments, the linear elastic and/or non-super-elastic nickel-titanium alloy may be in the range of about 50 to about 60 weight percent nickel, with the remainder being essentially titanium. In some embodiments, the composition is in the range of about 54 to about 57 weight percent nickel. One example of a suitable nickel-titanium alloy is FHP-NT alloy commercially available from Furukawa Techno Material Co. of Kanagawa, Japan. Other suitable materials may include ULTANIUM^{™} (available from Neo-Metrics) and GUM METAL^{™} (available from Toyota). In some other embodiments, a superelastic alloy, for example a superelastic nitinol can be used to achieve desired properties.

In at least some embodiments, portions or all of the system and/or other elements disclosed herein may also be doped with, made of, or otherwise include a radiopaque material. Radiopaque materials are understood to be materials capable of producing a relatively bright image on a fluoroscopy screen or another imaging technique during a medical procedure. This relatively bright image aids a user in determining the location and/or orientation of the system and/or other elements disclosed herein. Some examples of radiopaque materials can include, but are not limited to, gold, platinum, palladium, tantalum, tungsten alloy, polymer material loaded with a radiopaque filler, and the like.

In some embodiments, a degree of Magnetic Resonance Imaging (MRI) compatibility is imparted into the system and/or other elements disclosed herein. For example, the system and/or components or portions thereof may be made of a material that does not substantially distort the image and create substantial artifacts (e.g., gaps in the image). Certain ferromagnetic materials, for example, may not be suitable because they may create artifacts in an MRI image. The system or portions thereof, may also be made from a material that the MRI machine can image. Some materials that exhibit these characteristics include, for example, tungsten, cobalt-chromium-molybdenum alloys (e.g., UNS: R44003 such as ELGILOY^{®}, PHYNOX^{®}, and the like), nickel-cobalt-chromium-molybdenum alloys (e.g., UNS: R44035 such as MP35-N^{®} and the like), nitinol, and the like, and others.

In some embodiments, the system and/or other elements disclosed herein may be made from or include a polymer or other suitable material. Some examples of suitable polymers may include polytetrafluoroethylene (PTFE), ethylene tetrafluoroethylene (ETFE), fluorinated ethylene propylene (FEP), polyoxymethylene (POM, for example, DELRIN^{®} available from DuPont), polyether block ester, polyurethane (for example, Polyurethane 85A), polypropylene (PP), polyvinylchloride (PVC), polyether-ester (for example, ARNITEL^{®} available from DSM Engineering Plastics), ether or ester based copolymers (for example, butylene/poly(alkylene ether) phthalate and/or other polyester elastomers such as HYTREL^{®} available from DuPont), polyamide (for example, DURETHAN^{®} available from Bayer or CRISTAMID^{®} available from Elf Atochem), elastomeric polyamides, block polyamide/ethers, polyether block amide (PEBA, for example available under the trade name PEBAX^{®}), ethylene vinyl acetate copolymers (EVA), silicones, polyethylene (PE), MARLEX^{®} high-density polyethylene, MARLEX^{®} low-density polyethylene, linear low density polyethylene (for example REXELL^{®}), polyester, polybutylene terephthalate (PBT), polyethylene terephthalate (PET), polytrimethylene terephthalate, polyethylene naphthalate (PEN), polyetheretherketone (PEEK), polyimide (PI), polyetherimide (PEI), polyphenylene sulfide (PPS), polyphenylene oxide (PPO), poly paraphenylene terephthalamide (for example, KEVLAR^{®}), polysulfone, nylon, nylon-12 (such as GRILAMID^{®} available from EMS American Grilon), perfluoro(propyl vinyl ether) (PFA), ethylene vinyl alcohol, polyolefin, polystyrene, epoxy, polyvinylidene chloride (PVdC), poly(styrene-b-isobutylene-b-styrene) (for example, SIBS and/or SIBS 50A), polycarbonates, ionomers, biocompatible polymers, other suitable materials, or mixtures, combinations, copolymers thereof, polymer/metal composites, and the like. In some embodiments the sheath can be blended with a liquid crystal polymer (LCP). For example, the mixture can contain up to about 6 percent LCP.

In some embodiments, the system and/or other elements disclosed herein may include a fabric material disposed over or within the structure. The fabric material may be composed of a biocompatible material, such a polymeric material or biomaterial, adapted to promote tissue ingrowth. In some embodiments, the fabric material may include a bioabsorbable material. Some examples of suitable fabric materials include, but are not limited to, polyethylene glycol (PEG), nylon, polytetrafluoroethylene (PTFE, ePTFE), a polyolefinic material such as a polyethylene, a polypropylene, polyester, polyurethane, and/or blends or combinations thereof.

In some embodiments, the system and/or other elements disclosed herein may include and/or be formed from a textile material. Some examples of suitable textile materials may include synthetic yarns that may be flat, shaped, twisted, textured, preshrunk or un-shrunk. Synthetic biocompatible yarns suitable for use in the present disclosure include, but are not limited to, polyesters, including polyethylene terephthalate (PET) polyesters, polypropylenes, polyethylenes, polyurethanes, polyolefins, polyvinyls, polymethylacetates, polyamides, naphthalene dicarboxylene derivatives, natural silk, and polytetrafluoroethylenes. Moreover, at least one of the synthetic yarns may be a metallic yarn or a glass or ceramic yarn or fiber. Useful metallic yarns include those yarns made from or containing stainless steel, platinum, gold, titanium, tantalum or a Ni-Co-Cr-based alloy. The yarns may further include carbon, glass or ceramic fibers. Desirably, the yarns are made from thermoplastic materials including, but not limited to, polyesters, polypropylenes, polyethylenes, polyurethanes, polynaphthalenes, polytetrafluoroethylenes, and the like. The yarns may be of the multifilament, monofilament, or spun types. The type and denier of the yarn chosen may be selected in a manner which forms a biocompatible and implantable prosthesis and, more particularly, a vascular structure having desirable properties.

In some embodiments, the system and/or other elements disclosed herein may include and/or be treated with a suitable therapeutic agent. Some examples of suitable therapeutic agents may include anti-thrombogenic agents (such as heparin, heparin derivatives, urokinase, and PPack (dextrophenylalanine proline arginine chloromethylketone)); anti-proliferative agents (such as enoxaparin, angiopeptin, monoclonal antibodies capable of blocking smooth muscle cell proliferation, hirudin, and acetylsalicylic acid); anti-inflammatory agents (such as dexamethasone, prednisolone, corticosterone, budesonide, estrogen, sulfasalazine, and mesalamine); antineoplastic/antiproliferative/anti-mitotic agents (such as paclitaxel, 5-fluorouracil, cisplatin, vinblastine, vincristine, epothilones, endostatin, angiostatin and thymidine kinase inhibitors); anesthetic agents (such as lidocaine, bupivacaine, and ropivacaine); anticoagulants (such as D-Phe-Pro-Arg chloromethyl ketone, an RGD peptide-containing compound, heparin, anti-thrombin compounds, platelet receptor antagonists, anti-thrombin antibodies, anti-platelet receptor antibodies, aspirin, prostaglandin inhibitors, platelet inhibitors, and tick antiplatelet peptides); vascular cell growth promoters (such as growth factor inhibitors, growth factor receptor antagonists, transcriptional activators, and translational promoters); vascular cell growth inhibitors (such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin); cholesterol-lowering agents; vasodilating agents; and agents which interfere with endogenous vasoactive mechanisms.

It should be understood that this disclosure is, in many respects, only illustrative. Changes may be made in details, particularly in matters of shape, size, and arrangement of steps, without exceeding the scope of the disclosure. This may include, to the extent that it is appropriate, the use of any of the features of one example embodiment being used in other embodiments. The scope of the present invention is, of course, defined in the appended claims.

## Claims

1. An implant (100) for occluding a left atrial appendage (50), comprising:
an expandable framework (110) configured to shift between a collapsed configuration and an expanded configuration;
wherein the expandable framework includes a proximal hub (112) and a distal hub (114);
wherein a longitudinal axis (102) of the expandable framework extends from the proximal hub to the distal hub; and
a radiopaque marker (130) positioned longitudinally between the proximal hub and the distal hub in the expanded configuration,
wherein the radiopaque marker includes a body portion (140), a proximal leg (142), a distal leg (144), a first lateral tab (146), and a second lateral tab (152);
wherein the body portion is disposed against an exterior surface of the expandable framework,
wherein the first lateral tab (146) and the second lateral tab (152) wrap around a portion of the expandable framework (110).

2. The implant of claim 1, wherein the radiopaque marker (130) is oriented longitudinally in the expanded configuration.

3. The implant of any one of claims 1-2, wherein the proximal leg (142) and the distal leg (144) extend radially inwardly toward an interior of the expandable framework (110).

4. The implant of any one of claims 1-3, further comprising an occlusive element (120) disposed over at least a portion of the expandable framework (110).

5. The implant of claim 4,
wherein the radiopaque marker (130) includes a flattened element (440) at least partially embedded within the occlusive element (120).

6. The implant of any one of claims 1-5, wherein the radiopaque marker (130) includes a plurality of radiopaque markers (132, 134, 136) spaced apart about a circumference of the expandable framework (110).

7. A system for occluding a left atrial appendage (50), comprising:
a delivery sheath (40) and core wire (30) slidably disposed within a lumen (42) of the delivery sheath; and
the implant (100) of any one of claim 1-6, wherein the implant is releasably securable to a distal end of the core wire.

8. The system of claim 7, wherein the radiopaque marker (130) is formed from a different material than the expandable framework (110).

9. The system of any one of claims 7-8, wherein the radiopaque marker (130) has a different density than the expandable framework (110).

10. The system of claim 7, wherein the radiopaque marker (130) constitutes a first radiopaque marker (132), the implant (100) further comprising:
a second radiopaque marker (134) positioned longitudinally between the proximal hub (112) and the distal hub (114) in the expanded configuration; and
a third radiopaque (136) marker positioned longitudinally between the proximal hub and the distal hub in the expanded configuration;
wherein the first radiopaque marker, the second radiopaque marker, and the third radiopaque marker define a plane for positioning the expandable framework (110) relative to an ostium (52) of the left atrial appendage (50) in the expanded configuration.

11. The system of claim 10, wherein the first, second, and third radiopaque markers (132, 134, 136) each includes a body portion (140), a proximal leg (142), and a distal leg (144);
wherein the body portion is disposed against an exterior surface of the expandable framework (110);
wherein the proximal leg and the distal leg extend radially inwardly toward an interior of the expandable framework;
wherein when the implant (100) is being moved into the lumen (42), the proximal leg engages the delivery sheath (40) to urge the expandable framework (110) radially inward and away from the delivery sheath.

12. The system of any one of claims 10-11, wherein the first, second, and third radiopaque markers (132, 134, 136) each define a lateral extent and a longitudinal extent, the longitudinal extent being greater than the lateral extent;
wherein the longitudinal extent is oriented longitudinally with respect to the expandable framework (110).

## Patentansprüche

1. Implantat (100) zum Verschließen eines linken Herzohrs (50), umfassend:
einen expandierbaren Rahmen (110), der dazu konfiguriert ist, zwischen einer kollabierten Konfiguration und einer expandierten Konfiguration zu wechseln;
wobei der expandierbare Rahmen eine proximale Nabe (112) und eine distale Nabe (114) aufweist;
wobei sich eine Längsachse (102) des expandierbaren Rahmens von der proximalen Nabe zu der distalen Nabe erstreckt; und
einen röntgendichten Marker (130), der in der expandierten Konfiguration in Längsrichtung zwischen der proximalen Nabe und der distalen Nabe positioniert ist,
wobei der röntgendichte Marker einen Körperabschnitt (140), einen proximalen Schenkel (142), einen distalen Schenkel (144), eine erste laterale Lasche (146) und eine zweite laterale Lasche (152) beinhaltet;
wobei der Körperabschnitt gegen eine Außenfläche des expandierbaren Rahmens angeordnet ist,
wobei sich die erste laterale Lasche (146) und die zweite laterale Lasche (152) um einen Abschnitt des expandierbaren Rahmens (110) wickeln.

2. Implantat nach Anspruch 1, wobei der röntgendichte Marker (130) in der expandierten Konfiguration in Längsrichtung ausgerichtet ist.

3. Implantat nach einem der Ansprüche 1-2, wobei sich der proximale Schenkel (142) und der distale Schenkel (144) radial nach innen zu einem Inneren des expandierbaren Rahmens (110) hin erstrecken.

4. Implantat nach einem der Ansprüche 1-3, ferner umfassend ein Verschlusselement (120), das über mindestens einem Abschnitt des expandierbaren Rahmens (110) angeordnet ist.

5. Implantat nach Anspruch 4, wobei der röntgendichte Marker (130) ein abgeflachtes Element (440) beinhaltet, das zumindest teilweise in das Verschlusselement (120) eingebettet ist.

6. Implantat nach einem der Ansprüche 1-5, wobei der röntgendichte Marker (130) mehrere röntgendichte Marker (132, 134, 136) beinhaltet, die um einen Umfang des expandierbaren Rahmens (110) beabstandet sind.

7. System zum Verschließen eines linken Herzohrs (50), umfassend:
eine Einführschleuse (40) und einen Kerndraht (30), der verschiebbar innerhalb eines Lumens (42) der Einführschleuse angeordnet ist; und
das Implantat (100) nach einem der Ansprüche 1-6, wobei das Implantat lösbar an einem distalen Ende des Kerndrahts befestigbar ist.

8. System nach Anspruch 7, wobei der röntgendichte Marker (130) aus einem anderen Material als der expandierbare Rahmen (110) ausgebildet ist.

9. System nach einem der Ansprüche 7-8, wobei der röntgendichte Marker (130) eine andere Dichte als der expandierbare Rahmen (110) aufweist.

10. System nach Anspruch 7, wobei der röntgendichte Marker (130) einen ersten röntgendichten Marker (132) darstellt, wobei das Implantat (100) ferner Folgendes umfasst:
einen zweiten röntgendichten Marker (134), der in der expandierten Konfiguration in Längsrichtung zwischen der proximalen Nabe (112) und der distalen Nabe (114) positioniert ist; und
einen dritten röntgendichten (136) Marker, der in der expandierten Konfiguration in Längsrichtung zwischen der proximalen Nabe und der distalen Nabe positioniert ist;
wobei der erste röntgendichte Marker, der zweite röntgendichte Marker und der dritte röntgendichte Marker in der expandierten Konfiguration eine Ebene zum Positionieren des expandierbaren Rahmens (110) relativ zu einem Ostium (52) des linken Herzohrs (50) definieren.

11. System nach Anspruch 10, wobei der erste, der zweite und der dritte röntgendichte Marker (132, 134, 136) jeweils einen Körperabschnitt (140), einen proximalen Schenkel (142) und einen distalen Schenkel (144) beinhalten;
wobei der Körperabschnitt an einer Außenfläche des expandierbaren Rahmens (110) angeordnet ist;
wobei sich der proximale Schenkel und der distale Schenkel radial nach innen zu einem Inneren des expandierbaren Rahmens hin erstrecken;
wobei, wenn das Implantat (100) in das Lumen (42) bewegt wird, der proximale Schenkel die Einführschleuse (40) in Eingriff nimmt, um den expandierbaren Rahmen (110) radial nach innen und von der Einführschleuse weg zu drücken.

12. System nach einem der Ansprüche 10-11, wobei der erste, der zweite und der dritte röntgendichte Marker (132, 134, 136) jeweils eine laterale Erstreckung und eine Längserstreckung definieren, wobei die Längserstreckung größer als die laterale Erstreckung ist;
wobei die Längserstreckung in Bezug auf den expandierbaren Rahmen (110) in Längsrichtung ausgerichtet ist.

## Revendications

1. Implant (100) destiné à occlure un appendice auriculaire gauche (50), comprenant :
une structure déployable (110) configurée pour passer entre une configuration repliée et une configuration déployée ;
la structure déployable comprenant un moyeu proximal (112) et un moyeu distal (114) ;
un axe longitudinal (102) de la structure déployable s'étendant du moyeu proximal au moyeu distal ; et
un marqueur radio-opaque (130) positionné longitudinalement entre le moyeu proximal et le moyeu distal en configuration déployée,
le marqueur radio-opaque comprenant une partie de corps (140), une patte proximale (142), une patte distale (144), une première languette latérale (146), et une deuxième languette latérale (152) ;
la partie de corps étant disposée contre une surface extérieure de la structure déployable, la première languette latérale (146) et la deuxième languette latérale (152) s'enroulant autour d'une partie de la structure déployable (110).

2. Implant selon la revendication 1, le marqueur radio-opaque (130) étant orienté longitudinalement dans la configuration déployée.

3. Implant selon l'une quelconque des revendications 1 et 2, la patte proximale (142) et la patte distale (144) s'étendant radialement vers l'intérieur en direction de l'intérieur de la structure déployable (110).

4. Implant selon l'une quelconque des revendications 1 à 3, comprenant en outre un élément occlusif (120) disposé sur au moins une partie de la structure déployable (110).

5. Implant selon la revendication 4, le marqueur radio-opaque (130) comprenant un élément aplati (440) au moins partiellement noyé dans l'élément occlusif (120).

6. Implant selon l'une quelconque des revendications 1 à 5, le marqueur radio-opaque (130) comprenant une pluralité de marqueurs radio-opaques (132, 134, 136) espacés les uns des autres autour d'une circonférence de la structure déployable (110).

7. Système destiné à occlure un appendice auriculaire gauche (50), comprenant :
une gaine de distribution (40) et un fil central (30) disposés de manière coulissante dans une lumière (42) de la gaine de distribution ; et
l'implant (100) selon l'une quelconque des revendications 1 à 6, l'implant pouvant être fixé de manière libérable à une extrémité distale du fil central.

8. Système selon la revendication 7, le marqueur radio-opaque (130) étant formé à partir d'un matériau différent de celui de la structure déployable (110).

9. Système selon l'une quelconque des revendications 7 et 8, le marqueur radio-opaque (130) ayant une densité différente de celle de la structure déployable (110).

10. Système selon la revendication 7, le marqueur radio-opaque (130) constituant un premier marqueur radio-opaque (132), l'implant (100) comprenant en outre :
un deuxième marqueur radio-opaque (134) positionné longitudinalement entre le moyeu proximal (112) et le moyeu distal (114) dans la configuration déployée ; et
un troisième marqueur radio-opaque (136) positionné longitudinalement entre le moyeu proximal et le moyeu distal en configuration déployée ;
le premier marqueur radio-opaque, le deuxième marqueur radio-opaque et le troisième marqueur radio-opaque définissant un plan pour positionner la structure déployable (110) par rapport à un ostium (52) de l'appendice auriculaire gauche (50) dans la configuration déployée.

11. Système selon la revendication 10, les premier, deuxième et troisième marqueurs radio-opaques (132, 134, 136) comprenant chacun une partie de corps (140), une patte proximale (142) et une patte distale (144) ;
la partie de corps étant disposée contre une surface extérieure de la structure déployable (110) ;
la patte proximale et la patte distale s'étendant radialement vers l'intérieur vers un intérieur de la structure déployable ;
lorsque l'implant (100) est déplacé dans la lumière (42), la patte proximale entre en contact avec la gaine d'application (40) pour pousser la structure déployable (110) radialement vers l'intérieur et à l'écart de la gaine d'application.

12. Système selon l'une quelconque des revendications 10 et 11, les premier, deuxième et troisième marqueurs radio-opaques (132, 134, 136) définissant chacun une étendue latérale et une étendue longitudinale, l'étendue longitudinale étant supérieure à l'étendue latérale ; l'étendue longitudinale étant orientée longitudinalement par rapport à la structure déployable (110).
